# EUROPEAN PATENT APPLICATION

(11) **EP 2 982 414 A1**
(43) Date of publication of application: **10.02.2016**
(21) Application number: 13880980.1
(22) Date of filing: 01.04.2013
(51) Int. Cl.: A61N 5/10

(54) **PARTICLE BEAM PROJECTION CHAMBER AND PARTICLE BEAM TREATMENT DEVICE**

(71) Applicant: Mitsubishi Electric Corporation, Chiyoda-ku Tokyo 100-8310 (JP)
(72) Inventor: TAJIRI, Shinya, Tokyo 100-8310 (JP); TAKAHASHI, Osamu, Tokyo 100-8310 (JP)
(74) Representative: Sajda, Wolf E.
(86) International application number: PCT/JP2013/059837
(87) International publication number: WO 2014/162396

(57) **Abstract**

The particle beam irradiation room of this invention is purported to keep within an allowable range, a positional displacement amount from a positioned state of a patient even when the patient is moved from an irradiation-room entrance to an irradiation area after positioned at other than a patient table of the irradiation area actually used at the radiation of the particle beam. The particle beam irradiation room (20) of the invention includes: an irradiation execution room (22) in which a patient table (2) having a detachable-attachable top board (5) and an actuation device (9) for actuating the top board (5) is mounted, and that is surrounded by a shielding wall (21); a passage (23) formed in the shielding wall (21) on its side where a pre-room (25) for positioning the patient (45) relative to the top board (5) is located, in a form that allows the patient (45) positioned relative to the top board (5) to pass straight to the irradiation execution room (22) by a transport apparatus (10); and a shielding door (24) at the irradiation-room entrance (30) located on a side of the passage (23) toward the pre-room (25), for shielding against a leakage radiation at the radiation of the particle beam so that the leakage radiation is kept at a management value or less.

## Description

### TECHNICAL FIELD

The present invention relates to an irradiation room of a particle beam therapy system used for cancer therapy and the like.

### BACKGROUND ART

In recent years, among particle beam therapy systems for the purpose of cancer therapy, there have been advanced development and/or construction of a cancer therapy system that utilizes protons or heavy ions (will be referred to, in particular, as a particle beam therapy system). As is well known, according to a particle beam therapy utilizing protons, heavy ions, etc., a cancer diseased site can be irradiated in a concentrated manner as compared to the conventional radiation therapy with an X-ray, a γ-ray, etc. and thus it is possible to perform the therapy without affecting normal cells.

In the particle beam therapy, it is important to highly accurately radiate a particle beam to a tumor site such as a cancer. For that purpose, during the particle beam therapy, the patient is fastened to a top board of a bad (patient table) in a treatment room (irradiation room) using a fastener, etc. in order not to shift his/her position relative to the bed. In order to accurately position the tumor site such as a cancer within a radiation exposure region of the particle beam, a setting such as a coarse placement of the patient with the aid of a laser pointer, etc. is performed and then, a precise positioning of the diseased site of the patient is performed using an X-ray image or the like. In general, setting works (works for position setting) of the patient before irradiation for preforming a highly accurate radiation of the particle beam require approx. 10 minutes. As compared with a time of approx. 5 minutes required for irradiation, twice the time is required for the setting works before irradiation, which results in impairing effective operation of the particle beam therapy system.

In Patent Document 1, there is described a method of performing setting and positioning in a room (pre-room) different to the treatment room (this will be referred to as pre-room positioning) in order to reduce the time required for setting the patient in the treatment room (irradiation room). In the method of Patent Document 1, firstly, in a standby room, a first patient is laid on a first bed followed by attaching a fastener, and a setting such as a coarse placement with a laser pointer is performed.

Then, the patient is transported, while being laid on the first bed, along a first transport pathway to a simulator room, and a precise positioning of the patient is performed. At this time, in the standby room from which the first patient has been transported out, a next patient is laid on a second bed followed by attaching a fastener, and a setting such as a coarse placement with a laser pointer is performed. Then, the first patient subjected to the precise positioning in the simulator room is transported, while being laid on the first bed, along a second transport pathway to an irradiation room, and radiation of the particle beam to the diseased site is executed.

In this way, the irradiation room is used only at the time of radiation of the particle beam, and when one patient is under therapy, the next patient can be subjected to a positioning work in the standby room or the simulator room that is a pre-room, so that the utilization efficiency of the particle beam therapy system utilizing a particle beam is enhanced.

### LIST OF CITATIONS

### PATENT DOCUMENT

- Patent Document 1:: Japanese Patent Application Laid-Open JP 2000-288 102 A (From Paragraph 0020 to Paragraph 0022, FIG. 1, FIG. 2)

### SUMMARY OF THE INVENTION

### PROBLEMS TO BE SOLVED BY THE INVENTION

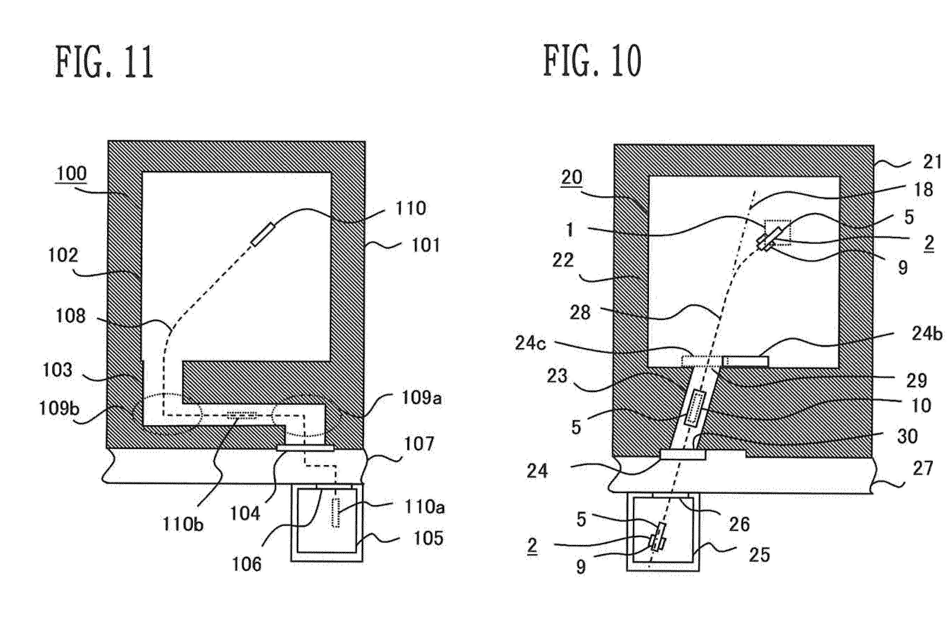

In the case of the method of setting a patient (patient-position setting) described in Patent Document 1, the patient positioned at each of the respective standby room and the simulator room is necessary to be moved, while keeping each positioned state, to the next room and finally to the irradiation room where a particle beam therapy is performed. In general, in order to be moved from an entrance of the irradiation room to a patient table mounted in the irradiation room, the patient passes through a labyrinth-like passage 103 as shown in FIG. 11 provided in consideration of shielding against the particle beam and its leakage radiation.

FIG. 11 is a diagram illustrating a conventional irradiation room and transportation of a patient. In FIG. 11, in an irradiation room 100 having a shielding door 104 and surrounded by a shielding wall 101, a patient bed 110 of Patent Document 1 is mounted. Although the patient bed 110 is single, numeral 110a is given to the patient bed at the time of positioning in a pre-room 105, and numeral 110b is given to the patient bed under transportation in the passage 103.

The patient is positioned at the patient bed 110a in the pre-room 105 having the pre-room door 106, and the patient fastened to the patient bed 110 is transported, while keeping the positioned state, through a corridor 107 and the passage 103 of the irradiation room 100 and along a transport pathway 108, to the location of the patient bed 110 in an irradiation execution room 102 of the irradiation room 100. As the conventional shielding door 104, since it is provided with the labyrinth-like passage 103, such a shielding door is used that is thin in thickness, namely, not so high in shielding effect to shield against the particle beam and the leakage radiation.

In the labyrinth-like passage 103, there are bent portions 109a, 109b where the direction is changed almost at a right angle. In Patent Document 1, it is assumed that the patient bed 110 (corresponding to the patient table) is moved while fastening the patient to the patient bed 110; however, no consideration is made for the labyrinth-like passage 103, and thus there is a problem that a positional displacement of the patient occurs when the direction is changed at the bent portion 109a or 109b of the passage 103.
If the positional displacement of the patient exceeds an allowable range, an adjustment in positioning is performed in the irradiation room 100, so that the working time in the irradiation room 100 for reproducing the positioned state becomes longer and thus, the utilization efficiency of the particle beam therapy system cannot be enhanced sufficiently.

This invention has been made to solve the problem as described above, and an object thereof is to provide a particle beam irradiation room by means of which an amount of positional displacement from the positioned state of the patient can be kept within the allowable range even when the patient is moved from the entrance of the irradiation room to an irradiation area thereof after being subjected to positioning at other than the patient table of the irradiation area actually used at the time of radiation of the particle beam.

### MEANS FOR SOLVING THE PROBLEMS

It is characterized by comprising: an irradiation execution room in which a patient table having a detachable-attachable top board and an actuation device for actuating the top board is mounted, and that is surrounded by a shielding wall; a passage that is formed in the shielding wall on its side where a pre-room in which another actuation device constituting another patient table for positioning a patient relative to the top board is mounted is located, in such a form that allows the patient positioned relative to the top board to pass straight to the irradiation execution room by way of a transport apparatus; and a shielding door at an irradiation-room entrance located on a side of the passage toward the pre-room, that shields against a leakage radiation at the time of radiation of the particle beam so that the leakage radiation is kept at a management value or less.

### EFFECT OF THE INVENTION

The particle beam irradiation room according to the invention comprises the passage formed in a form straight-passable to the irradiation execution room, and the shielding door at an irradiation-room entrance that shields against a leakage radiation at the time of radiation of the particle beam so that the leakage radiation is kept at a management value or less, and thus, an amount of positional displacement from the positioned state of the patient can be kept within an allowable range even when the patient is moved from the entrance of the irradiation room to the irradiation area after being subjected to positioning at other than the patient table of the irradiation area actually used at the time of radiation of the particle beam.

Since an amount of the positional displacement at the time of movement to the irradiation area can be kept within an allowable range, the working time in the irradiation room for reproducing the positioned state can be made shorter, so that the utilization efficiency of the particle beam therapy system can be enhanced sufficiently.

### BRIEF DESCRIPTION OF THE DRAWINGS

- FIG. 1: is a diagram showing a particle beam irradiation room according to Embodiment 1 of the invention.
- FIG. 2: is a diagram illustrating an attitude of a patient at the time of transportation of the patient according to Embodiment 1 of the invention.
- FIG. 3: is a schematic configuration diagram of a particle beam therapy system provided with a particle beam irradiation room of the invention.
- FIG. 4: is a diagram showing a configuration of the particle beam irradiation apparatus in FIG. 3.
- FIG. 5: is a diagram showing a patient-position setting system of the invention.
- FIGS. 6A and 6B: are diagrams showing a patient table in FIG. 5.
- FIG. 7: is a diagram showing a transport apparatus in FIG. 5.
- FIG. 8: is a diagram showing another particle beam irradiation room according to Embodiment 1 of the invention.
- FIG. 9: is a diagram showing a particle beam irradiation room according to Embodiment 2 of the invention.
- FIG. 10: is a diagram showing another particle beam irradiation room according to Embodiment 2 of the invention.
- FIG. 11: is a diagram illustrating a conventional irradiation room and transportation of a patient.

### MODES FOR CARRYING OUT THE INVENTION

### Embodiment 1

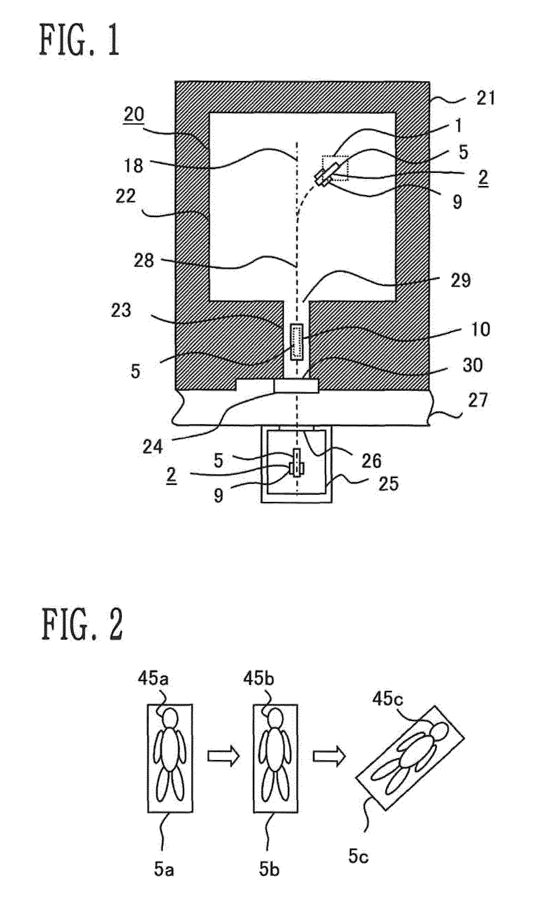

A particle beam irradiation room and particle beam irradiation apparatus provided with the particle beam irradiation room of the invention will be described. FIG. 1 is a diagram showing a particle beam irradiation room according to Embodiment 1 of the invention, and FIG. 2 is a diagram illustrating an attitude of a patient at the time of transportation of the patient according to Embodiment 1 of the invention.

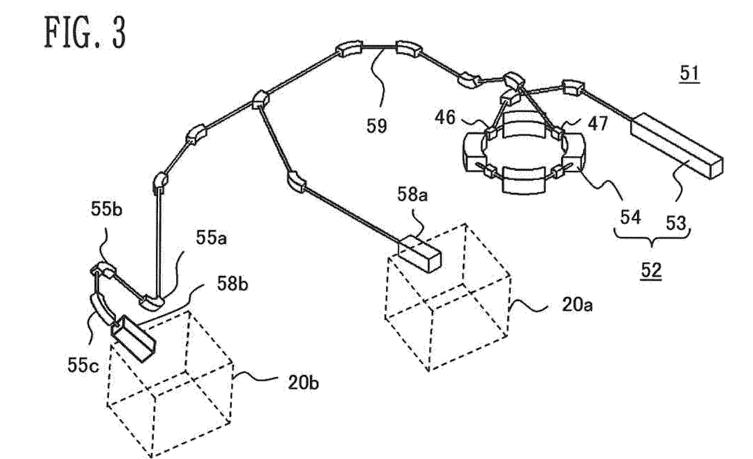

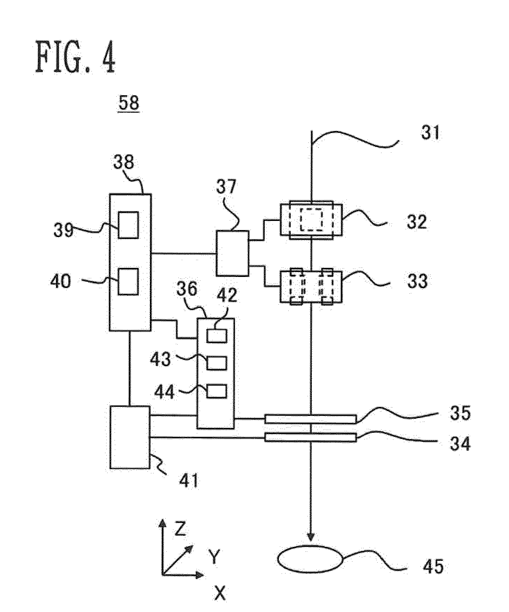

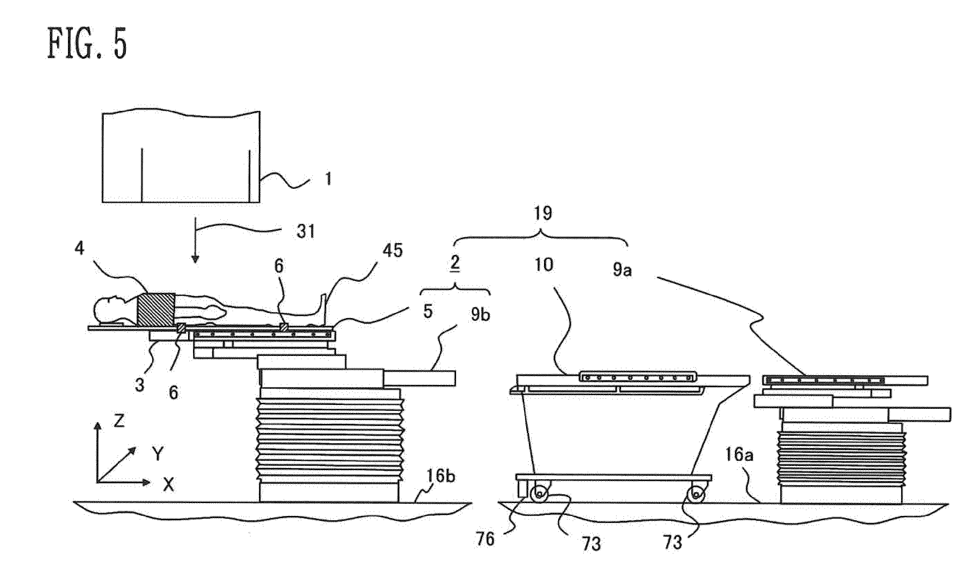

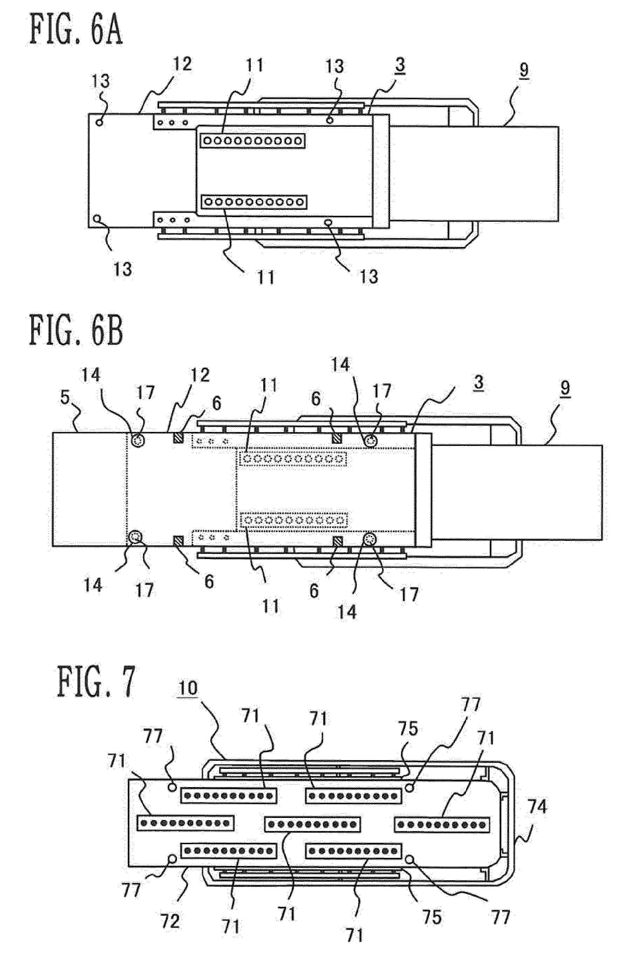

FIG. 3 is a schematic configuration diagram of a particle beam therapy system provided with a particle beam irradiation room of the invention, and FIG. 4 is a diagram showing a configuration of the particle beam irradiation apparatus in FIG. 3. FIG. 5 is a diagram showing a patient-position setting system of the invention. FIGS. 6A and 6B are diagrams showing a patient table in FIG. 5, and FIG. 7 is a diagram showing a transport apparatus in FIG. 5.

A particle beam irradiation room 20 (referred to as an irradiation room, if appropriate) according to Embodiment 1 of the invention includes an irradiation execution room 22 that are surrounded by a shielding wall 21 and a straight-passable passage 23, and a shielding door 24 placed at an irradiation-room entrance 30. In the irradiation room 20, there are placed a patient table 2 and an irradiation nozzle 1 that constitutes a downstream side of a particle beam irradiation apparatus 58 and that radiates a charged particle beam 31 to a patient 45 laid on the patient table 2.

The patient 45 is positioned at another patient table 2 mounted in a pre-room 25 having a pre-room door 26, and then, the patient 45 fastened to a top board 5 is transported, while keeping the positioned state, through a corridor 27, the irradiation-room entrance 30, the passage 23 of the irradiation room 20 and an irradiation-execution-room entrance 29 and along a transport pathway 28, to the location of the patient bed 2 in the irradiation execution room 22 in the irradiation room 20.

A shielding wall 24 is thicker in thickness than the shielding wall 104 placed in the conventional irradiation room, namely, it has a higher shielding effect to shield against the particle beam. During a particle beam therapy, there is a leakage radiation toward other than the diseased site. During a particle beam therapy, a secondary radiation is produced because the particle beam passes a window of the irradiation nozzle 1 or the like, or passes inside the body of the patient 45.

The secondary radiation is also a leakage radiation. The thickness and the material of the shielding wall 24 are selected so that the leakage radiation produced at the time of actually radiating a particle beam (the charged particle beam 31, etc.) in the irradiation room 20 can be kept at a predetermined management value or less. The predetermined management value is, for example, a legal value defined by a law.

The patient-position setting system described in Japanese Patent Application Laid-Open JP 2012-148 026 A can be used as a patient-position setting system 19 for performing positioning of the patient at other than the patient table 2 of the irradiation area actually used at the time of radiation of the particle beam, for moving the patient 45 while keeping the positioned state of the patient relative to the top board 5 of the patient table 2 to the irradiation area, and for reproducing the positioned state of the patient 45 in the irradiation area.

The patient-position setting system 19 includes, as shown in FIG. 5: the patient table 2 having the top board 5 on which the patient 45 is laid and an actuation device 9b for actuating the top board 5; a transport apparatus 10 for transporting the top board 5; and an actuation device 9a for constituting another patient table 2. For the actuation devices, numeral 9 is used collectively, and numerals 9a, 9b are used when they are to be described distinctively. The detachable-attachable top board 5 and the actuation device 9 constitute the patient tables 2.

The particle beam irradiation room 20 of Embodiment 1 is provided with the straight-passable passage 23 and the shielding wall 24 that is thicker in thickness than the shielding wall 104 placed in the conventional irradiation room 100, namely, that has a higher shielding effect to shield against the particle beam and the leakage radiation, so that the patient 45 laid on the top board 5 can be transported almost straight in the straight-passable passage 23, and the leakage radiation produced during a particle beam therapy can be kept at a predetermined management value or less.

In FIG. 1, an example is shown in which the passage 23 is placed perpendicular to the corridor 27 and the pre-room 25 is placed so that the center line 18 of the passage 23 passes therethrough. Note that although the center line 18 is overlapped with the transport pathway 28, for avoiding complexity in FIG. 1, it is only shown at near the two patient tables 2. The same will apply to the other figures showing the center line 18.

Using FIG. 3 and FIG. 4, a particle beam therapy system 51 and the particle beam irradiation apparatus 58 will be described. The particle beam therapy system 51 includes a beam generation apparatus 52, a beam transport system 59, particle beam irradiation apparatuses 58a, 58b and particle beam irradiation rooms 20a, 20b. The beam generation apparatus 52 has an ion source (not shown), a pre-accelerator 53 and a charged particle accelerator 54. The particle beam irradiation apparatus 58b is placed in a rotary gantry (not shown), and the irradiation nozzle of the particle beam irradiation apparatus 58b is placed in the particle beam irradiation room 20b.

The particle beam irradiation apparatus 58a is placed in the particle beam irradiation room 20a having no rotary gantry. The role of the beam transport system 59 is to communicate between the charged particle accelerator 54 and the particle beam irradiation apparatuses 58a, 58b. The beam transport system 59 is partly placed in the rotary gantry (not shown) and has, at that part, a plurality of deflection electromagnets 55a, 55b, 55c.

The charged particle beam 31 that is a particle beam, such as a proton beam, etc., generated by the ion source, is accelerated by the pre-accelerator 53 and injected into the charged particle accelerator 54 through an injection device 46. The charged particle accelerator 54 is a synchrotron, for example. The charged particle beam 31 is accelerated up to a given energy. The charged particle beam 31 emitted from an emission device 47 of the charged particle accelerator 54, is transported through the beam transport system 59 to the particle beam irradiation apparatuses 58a, 58b.

The particle beam irradiation apparatuses 58a, 58b each radiate the charged particle beam 31 to the diseased site of the patient 45. For the particle beam irradiation apparatuses, numeral 58 is used collectively, and numerals 58a, 58b are used when they are to be described distinctively. For the particle beam irradiation rooms, numeral 20 is used collectively, and numerals 20a, 20b are used when they are to be described distinctively.

The charged particle beam 31 generated by the beam generation apparatus 52 and accelerated up to the given energy, is brought through the beam transport system 59 to the particle beam irradiation apparatus 58. In FIG. 4, the particle beam irradiation apparatus 58 includes: an X-direction scanning electromagnet 32 and a Y-direction scanning electromagnet 33 which scan the charged particle beam 31, respectively in an X-direction and a Y-direction that are directions perpendicular to the charged particle beam 31; a position monitor 34; a dose monitor 35; a dose-data converter 36; a beam-data processing device 41; a scanning-electromagnet power source 37; and an irradiation management device 38 for controlling the particle beam irradiation apparatus 58.

The irradiation management device 38 includes an irradiation control computer 39 and an irradiation control device 40. The dose-data converter 36 includes a trigger generation unit 42, a spot counter 43 and an inter-spot counter 44. Note that in FIG. 4, the travelling direction of the charged particle beam 31 is a direction of -Z.

The X-direction scanning electromagnet 32 is a scanning electromagnet for scanning the charged particle beam 31 in the X-direction, and the Y-direction scanning electromagnet 33 is a scanning electromagnet for scanning the charged particle beam 31 in the Y-direction. With respect to the charged particle beam 31 scanned by the X-direction scanning electromagnet 32 and the Y- direction scanning electromagnet 33, the position monitor 34 detects beam information for calculating a passing position (gravity center position) and a size of the beam that passes therethrough.

The beam-data processing device 41 calculates the passing position (gravity center position) and the size of the charged particle beam 31 on the basis of the beam information that comprises a plurality of analog signals detected by the position monitor 34. Further, the beam-data processing device 41 generates an abnormality detection signal indicative of a position abnormality and/or a size abnormality of the charged particle beam 31, and outputs the abnormality detection signal to the irradiation management device 38.

The dose monitor 35 detects the dose of the charged particle beam 31. The irradiation management device 38 controls the irradiation position of the charged particle beam 31 in the diseased site of the patient 45 on the basis of treatment plan data prepared by an unshown treatment plan device, and moves the charged particle beam 31 to a next irradiation position when the dose having been measured by the dose monitor 35 and converted by the dose-data converter 36 into digital data, reaches a target dose.

The scanning-electromagnet power source 37 changes setup currents for the X-direction scanning electromagnet 32 and the Y-direction scanning electromagnet 33 on the basis of control inputs (commands) output from the irradiation management device 38 for the X-direction scanning electromagnet 32 and the Y-direction scanning electromagnet 33.

Here, the scanning irradiation method of the particle beam irradiation apparatus 58 is assumed to be a raster-scanning irradiation method in which the charged particle beam 31 is not stopped when the irradiation position of the charged particle beam 31 is changed, that is a method in which the beam irradiation position moves between spot positions successively like a spot-scanning irradiation method. The spot counter 43 serves to measure an amount of irradiation dose during when the beam irradiation position of the charged particle beam 31 is staying.

The inter-spot counter 44 serves to measure an amount of irradiation dose during when the beam irradiation position of the charged particle beam 31 is moving. The trigger generation unit 42 serves to generate a dose completion signal when the dose of the charged particle beam 31 at a beam irradiation position reaches the target irradiation dose.

Using FIG. 5, FIGS. 6A, 6B and FIG. 7, the patient-position setting system 19 will be described. FIG. 6A is a top view of the actuation device 9 in the patient table 2, and FIG. 6B is a top view of the patient table 2, namely, a top view when the top board 5 is mounted on the actuation device 9. FIG. 7 is a top view of the transport apparatus 10. The patient table 2 has the top board 5 on which the patient 45 is laid, and the actuation device 9 for actuating the top board 5. A top-board support portion 3 of the actuation device 9 supports the top board 5.

The top-board support portion 3 is configured by an upper part of the body of the actuation device 9 and a top-board support plate 12. In the top-board support portion 3, a plurality of air-driven ball bearings 11 and a plurality of top-board fixing holes 13 are provided. In FIGS. 6A, 6B, an example is shown in which two air-driven ball bearings 11 and four top-board fixing holes 13 are present.

The air-driven ball bearing 11 is a product generally used in a production line or the like, which can supports the top board 5 by balls that are lifted up by high pressure air supplied thereto, to thereby allow a transport object to move freely due to rotation of the balls. In normal time when high pressure air is not supplied, the air-driven ball bearing 11 is allowed so that its ball portion is lower than the top surface of the actuation device 9. By uplifting the balls only when necessary so that the slide friction resistance between the top-board support portion 3 and the top board 5 during rotation of the balls is reduced, the air-driven ball bearing 11 can allow the top board 5 to move freely.

In the transport apparatus 10, a seven number, for example, of ball bearings 71 and a four number, for example, of top-board fixing holes 77 are provided on a transport-apparatus upper surface 72 on which the top board 5 is placed. At both sides in transverse direction of the transport-apparatus upper surface 72, guides 75 are provided. A handrail 74 is provided along the circumference of the transport apparatus 10, and at a lower part of the transport apparatus 10, four casters 73 and a fixing stopper 76 (see, FIG. 5) are provided. The fixing stopper 76, when pressed to contact with a floor 16a or 16b, fixes the transport apparatus 10.

As differs from the air-driven ball bearing 11, the ball portion of the ball bearing 71 is higher than the transport-apparatus upper surface 72. Similarly to the air-driven ball bearing 11, the ball bearing 71 can allow the top board 5 to move freely when the slide friction resistance between the top-board support portion 3 and the top board 5 during rotation of the balls is reduced. Because of the guides 75 provided at both sides in transverse direction of the transport-apparatus upper surface 72, when the top board 5 is to be moved to the transport apparatus 10 or when the top board 5 is to be dropped off from the transport apparatus 10, it is possible to guide the top board in a predetermined direction (a longitudinal direction). Note that the floor 16a is a floor in the pre-room 25, and the floor 16b is a floor in the irradiation room 20.

At the positioning between the actuation device 9 and the top board 5, the plurality of top-board fixing holes 13 provided in the top-board support portion 3 and a plurality of fixing holes for positioning 17 provided in the top board 5 are used. The fixing holes for positioning 17 are through holes. The fixing holes for positioning 17 are each formed in such a manner that it can encompass each of the top-board fixing holes 13 as viewed from the upper-surface side of the top board 5.

The position determining portion in the actuation device 9 is the top-board fixing holes 13 and the position determining portion in the top board 5 is the fixing holes for positioning 17. Pins 14 are inserted into the top-board fixing holes 13 and the fixing holes for positioning 17. For example, a shaft portion of each of the pin 14 has a taper, so that by pushing the pins 14 into the top-board fixing holes 13 and the fixing holes for positioning 17, the top-board fixing holes 13 and the fixing holes for positioning 17 are positioned in a predetermined positional relationship therebetween.

In this way, by the top-board fixing holes 13 and the fixing holes for positioning 17 that are position determining portions, the top board 5 is positioned at a predetermined position on the top-board support portion 3. Thereafter, as shown in FIG. 5 and FIG. 6B, by use of top-board fixtures 6, the top-board support portion 3 and the top board 5 are fixed, for example, in a clamped manner.

A patient-position setting method will be described. In the pre-room 25, the patient 45 is laid on the top board 5 and is then subjected to a highly accurate positioning by using a CT system or an X-ray radiographic system and by driving the actuation device 9 so that a CT image or an X-ray image is matched to a reference image for positioning (Position Information Determination Step). The patient 45 is laid on the top board 5 of the patient table 2 followed by attaching a patient fastener 4, and then, a setting such as a coarse placement of the patient with a laser pointer is performed.

Subsequently, the patient 45 is subjected to such a precise positioning. In the pre-room 25, the CT system or the X-ray radiographic system is placed. Using a CT image of the CT system or an X-ray image of the X-ray radiographic system, the patient is subjected to the positioning by driving motors for making settings in respective directions of axes of the actuation device 9 so that the image is matched to the diseased site in a reference CT image used when a treatment plan was prepared.

Actuation-device parameters of the patient table 2 that are determined from the positioning in the pre-room 25 are transferred through a network to the patent table 2 in the irradiation room 20. In FIG. 2, a top board 5a and a patient 45a show an attitude of the patient highly accurately positioned in the pre-room 25. Note that the up-down direction in FIG. 2 corresponds to the direction of the center line 18 in the transport pathway 28.

Here, the positioning by the patient table 2 in the pre-room 25 is performed with reference to an isocenter of the patient table 2 in the irradiation room 20. Namely, the actuation-device parameters of the patient table 2 in the pre-room 25 have been adjusted so that a virtual isocenter on the top board 5 in the patient table 2 in the pre-room 25 is matched to the isocenter on the top board 5 in the patient table 2 in the irradiation room 20; or with respect to the actuation-device parameters of the patient table 2 in the pre-room 25, the data of the actuation-device parameters to be transferred will be offset-adjusted so that a virtual isocenter on the top board 5 in the patient table 2 in the pre-room 25 is matched to the isocenter on the top board 5 in the patient table 2 in the irradiation room 20.

Then, the patient 45 is transported, while keeping his/her fastened state to the top board 5, with the top board 5 by the transport apparatus 10 (Patient Transport Step). The top board 5 to which the patient 45 is fastened by the patient fastener 4, is transported by being placed on the transport apparatus 10 to the irradiation area of the irradiation room 20.

Specifically, by way of the transport apparatus 10, the patient 45 laid on the top board 5 is passed through the passage 23 of the irradiation room 20 and is transported to the irradiation area in the irradiation execution room 22 of the irradiation room 20 where the actuation device 9 is placed. Since the passage 23 is placed in a straight passable form, the patient 45 laid on the top board 5 is transported almost straight in the passage 23 to reach the irradiation execution room 22. Even after entering the irradiation execution room 22, the patient 45 laid on the top board 5 is transported almost straight along the center line 18 of the passage 23, for example, until the vicinity of the center of the irradiation execution room 22.

Thereafter, the patient 45 laid on the top board 5 is transported, while changing the direction by a gentle angle to be directed to the actuation device 9, to the irradiation area where the actuation device 9 is placed. In FIG. 1, there are shown the top board 5 and the transport apparatus 10 in the passage 23 that are in the course of transportation, and a top board 5b and a patient 45b in FIG. 2 show an attitude of the patient in the passage 23 who is in the course of transportation.

Then, the patient 45 is moved, while keeping the fastened state to the top board 5, to the actuation device 9 in the irradiation room 20, so that the positioned state at the patient table 2 in the pre-room 25 is reproduced (Positioned State Reproduction Step). While being in the fastened state to the top board 5, the patient 45 is placed from the transport apparatus 10 onto the top-board support portion 3 of the actuation device 9 in the irradiation room 20, so that the relative positions of the top board 5 and the top-board support portion 3 are reproduced by the position determining portions provided in the top-board support portion 3 and the top board 5.

The top board 5 is fixed to the top-board support portion 3 of the actuation device 9 by use of a plurality of top-board fixtures 6. Thereafter, the actuation device 9 acquires the actuation-device parameters of the patient table 2 used in the pre-room 25 for positioning. The actuation device 9 drives the motors for making settings in the respective directions of the axes so that the top board 5 and the patient 45 are reproduced into their positional condition having been highly accurately positioned in the pre-room 25.

After the positioned state has been confirmed using an X-ray radiographic system and the positioned state is reproduced, the charge particle beam 31 is radiated from the irradiation nozzle 1 of the charged particle irradiation apparatus 58 to the diseased site of the patient 45. In FIG. 2, a top board 5c and a patient 45c show an attitude of the patient whose positioned state is reproduced in the irradiation room 20, which is inclined from the center line 18 in the transport pathway 28.

The particle beam irradiation room 20 of Embodiment 1 is provided with the straight-passable passage 23, and the shielding door 24 that is higher in shielding effect to shield against the particle beam and the leakage radiation than the shielding door 104 placed in the conventional irradiation room 100. Thus, the patient 45 laid on the top board 5 can be transported almost straight in the straight-passable passage 23, as well as the leakage radiation produced during the particle beam therapy can be kept at a predetermined management value or less.

Further, according to the particle beam irradiation room 20 of Embodiment 1, the bent portions 109a, 109b in the conventional labyrinth-like passage 103 can be eliminated, so that the transport pathway 28 for transporting the patient 45 can be reduced relative to the conventional transport pathway 108 because of the straight-passable passage 23. The reduction in the transport pathway makes it possible to reduce the moving distance for movement of the patient 45, to thereby shorten the patient transport time.

Shortening of the patient transport time makes it possible to reduce the possibility that the patient 45 causes a displacement in the course of transportation. It is preferable that the length of the straight-passable passage 23 be as short as possible. The passage 23 shown in FIG. 1 is most advantageous, since its center line 18 is placed perpendicular to the corridor 27, to reduce the transport pathway in the passage 23.

The particle beam therapy system 51 provided with the particle beam irradiation room 20 of Embodiment 1 can perform position setting of the patient 45 by way of the patient-position setting system 19 in such a manner that the patient 45 is subjected to positioning at other than the patient table 2 in the irradiation room 20 actually used at the time of radiation of the particle beam 31, and is then moved to the irradiation room 20 while keeping the state of the patient 45 positioned relative to the top board 5 of the patient table 2, followed by reproduction of the positioned state of the patient 45 in the irradiation room 20.

Thus, it is possible to largely shorten the position setting work of the patient 45 performed in the irradiation room 20. Further, according to the particle beam therapy system 51 provided with the particle beam irradiation room 20 of Embodiment 1, the patient 45 laid on the top board 5 can be transported almost straight by the straight-passable passage 23, so that an amount of positional displacement from the positioned state of the patient 45 can be kept within an allowable range even when the patient is moved from the entrance of the irradiation room 20 (irradiation-room entrance 30) to the irradiation area.

Since the particle beam therapy system 51 provided with the particle beam irradiation room 20 of Embodiment 1 can keep an amount of the positional displacement within an allowable range at the time of movement to the irradiation area, the working time in the irradiation room 20 for reproducing the positioned state can be made shorter, so that the utilization efficiency of the particle beam therapy system 51 can be enhanced sufficiently.

According to the particle beam irradiation room 20 of Embodiment 1, it comprises: the irradiation execution room 22 in which the patient table 2 having the detachable-attachable top board 5 and the actuation device 9 for actuating the top board 5, is mounted, and that is surrounded by the shielding wall 21; the passage 23 that is formed in the shielding wall 21 on its side where the pre-room 25 in which the other actuation device 9 constituting the other patient table 2 for positioning the patient 45 relative to the top board 5 is mounted, is located, in such a form that allows the patient 45 positioned relative to the top board 5 to pass straight to the irradiation execution room 22 by way of the transport apparatus 10; and the shielding door 24 at the irradiation-room entrance 30 located on a side of the passage 23 toward the pre-room 25, that shields against a leakage radiation at the time of radiation of the particle beam (charged particle beam 31, etc.) so that the leakage radiation is kept at a management value or less.

Thus, an amount of positional displacement from the positioned state of the patient 45 can be kept within an allowable range even when the patient 45 is moved from the entrance of the irradiation room 20 (irradiation-room entrance 30) to the irradiation area after being subjected to positioning at other than the patient table 2 of the irradiation table actually used at the time of radiation of the particle beam (charged particle beam 31, etc.). Since an amount of the positional displacement at the time of movement to the irradiation area can be kept within an allowable range, the working time in the irradiation room 20 for reproducing the positioned state can be made shorter, so that the utilization efficiency of the particle beam therapy system 51 can be enhanced sufficiently.

According to the particle beam therapy system 51 of Embodiment 1, it comprises: the beam generation apparatus 52 that generates the charged particle beam 31 and accelerates the charged particle beam up to a given energy by using the accelerator (charged particle accelerator 54); the beam transport system 59 that transports the charged particle beam 31 accelerated by the beam generation apparatus 52; the particle beam irradiation apparatus 58 that radiates the charged particle beam 31 transported by the beam transport system 59 to the patient 45; and the particle beam irradiation room 20 in which the irradiation nozzle 1 that constitutes a downstream side of the particle beam irradiation apparatus 58 is placed.

The particle beam irradiation room 20 provided in the particle beam therapy system 51 of Embodiment 1 comprises: the irradiation execution room 22 in which the patient table 2 having the detachable-attachable top board 5 and the actuation device 9 for actuating the top board 5, is mounted, and that is surrounded by the shielding wall 21; the passage 23 that is formed in the shielding wall 21 on its side where the pre-room 25 in which the other actuation device 9 constituting the other patient table 2 for positioning the patient 45 relative to the top board 5 is mounted, is located, in such a form that allows the patient 45 positioned relative to the top board 5 to pass straight to the irradiation execution room 22 by way of the transport apparatus 10; and the shielding door 24 at the irradiation-room entrance 30 located on a side of the passage 23 toward the pre-room 25, that shields against a leakage radiation at the time of radiation of the particle beam (charged particle beam 31, etc.) so that the leakage radiation is kept at a management value or less.

Thus, because of the patient being transported through the straight-passable passage 23 by the patient-position setting system 19, an amount of the positional displacement from the positioned state of the patient 45 can be kept within an allowable range even when the patient is moved from the entrance of the irradiation room 20 (irradiation-room entrance 30) to the irradiation area, so that the working time in the irradiation room 20 for reproducing the positioned state can be made shorter and thus the utilization efficiency of the particle beam therapy system 51 can be enhanced sufficiently.

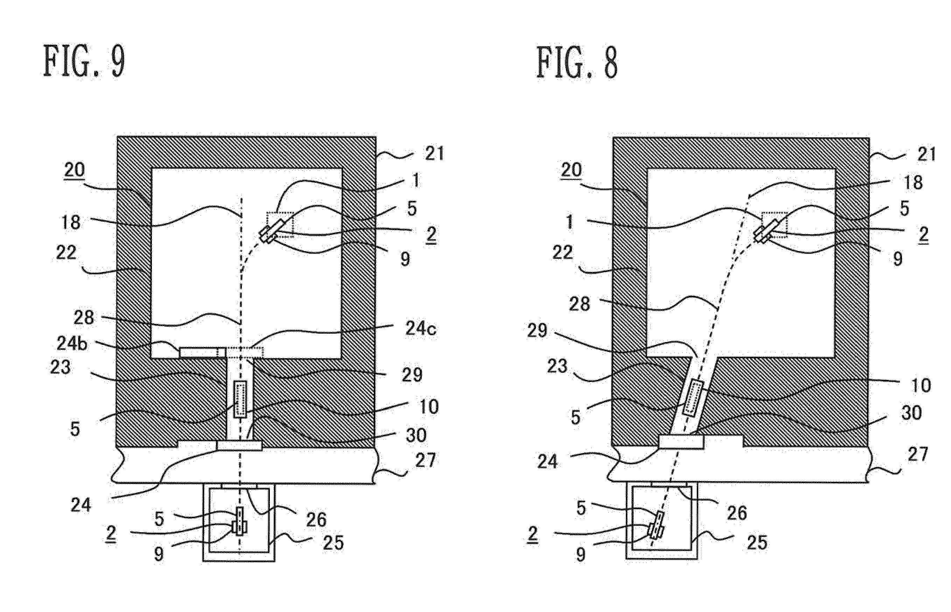

In FIG. 1, an example is shown in which the passage 23 is placed perpendicular to the corridor 27 and the pre-room 25 is placed so that the center line 18 of the passage 23 passes therethrough; however, as shown in FIG. 8, another straight-passable passage 23 may instead be placed. FIG. 8 is a diagram showing another particle beam irradiation room according to Embodiment 1 of the invention.

In FIG. 8, an example is shown in which the passage 23 is placed obliquely to the corridor 27 and the pre-room 25 is placed so that the center line 18 of the passage 23 passes therethrough. The particle beam irradiation room 20 shown in FIG. 8 also provides the same effect as that by the particle beam irradiation room 20 shown in FIG. 1.

### Embodiment 2

FIG. 9 is a diagram showing a particle beam irradiation room according to Embodiment 2 of the invention. The particle beam irradiation room 20 of Embodiment 2 differs from the particle beam irradiation room 20 of Embodiment 1 in that a shielding door 24 is provided also at the irradiation-execution-room entrance 29.

In FIG. 9, numeral 24b is given to the shielding door at the time of transportation of the patient 45 laid on the top board 5, and numeral 24c is given to the shielding door at the time of radiation of the particle beam.

In Embodiment 2, there is placed a plurality of shielding doors 24, so that the plurality of shielding doors are each allowed to have a lower shielding effect as compared with the case of shielding only by one of them against the leakage radiation produced during the particle beam therapy. Accordingly, restrictions in design of them can be reduced as compared with the shielding door 24 of Embodiment 1, so that it is possible to prepare these shielding doors 24 more easily than the shielding door 24 of Embodiment 1.

FIG. 10 is a diagram showing another particle beam irradiation room according to Embodiment 2 of the invention. What is different from the other particle beam irradiation room 20 of Embodiment 1 is that a shielding door 24 is provided also at the irradiation-execution-room entrance 29.

In FIG. 10, an example is shown in which the passage 23 is placed obliquely to the corridor 27 and the pre-room 25 is placed so that the center line 18 of the passage 23 passes therethrough. The particle beam irradiation room 20 shown in FIG. 10 also provides the same effect as that by the particle beam irradiation room 20 shown in FIG. 9.

It should be noted that unlimited combination of the respective embodiments and any appropriate modification or omission in the embodiments may be made in the present invention without departing from the scope of the invention.

### DESCRIPTION OF REFERENCE NUMERALS and SIGNS

- 1: irradiation nozzle,
- 2: patient table
- 5, 5a: top board
- 5b, 5c: top board
- 9, 9a, 9b: actuation devices,
- 10: transport apparatus
- 18: center line
- 20, 20a, 20b: particle beam irradiation rooms
- 21: shielding wall
- 22: irradiation execution room
- 23: passage
- 24, 24b, 24c: shielding doors
- 25: pre-room
- 29: irradiation-execution -room entrance
- 30: irradiation-room entrance
- 31: charged particle beam
- 45, 45a: patient
- 45b, 45c: patient
- 51: particle beam therapy system
- 52: beam generation apparatus
- 54: charged particle accelerator
- 58, 58a, 58b: particle beam irradiation apparatus
- 59: beam transport system.

## Claims

1. A particle beam irradiation room having a patient table mounted therein on which a patient is laid at the time of radiation of a particle beam,
wherein the patient table has a detachable-attachable top board and an actuation device for actuating the top board;
the particle beam irradiation room comprising:
- an irradiation execution room in which the patient table is mounted and that is surrounded by a shielding wall;
- a passage that is formed in the shielding wall on its side where a pre-room in which another actuation device constituting another patient table for positioning the patient relative to the top board is mounted, is located, in such a form that allows the patient positioned relative to the top board to pass straight to the irradiation execution room by way of a transport apparatus; and
- a shielding door at an irradiation-room entrance located on a side of the passage toward the pre-room, that shields against a leakage radiation at the time of radiation of the particle beam so that the leakage radiation is kept at a management value or less.

2. The particle beam irradiation room of claim 1,
further comprising another shielding door at an irradiation-execution-room entrance on a side of the passage toward the irradiation execution room.

3. A particle beam therapy system comprising:
- a beam generation apparatus that generates a charged particle beam and accelerates the charged particle beam up to a given energy by using an accelerator;
- a beam transport system that transports the charged particle beam accelerated by the beam generation apparatus;
- a particle beam irradiation apparatus that radiates the charged particle beam transported by the beam transport system to a patient; and a particle beam irradiation room in which an irradiation nozzle that constitutes a downstream side of the particle beam irradiation apparatus is placed; wherein the particle beam irradiation room is the particle beam irradiation room of claim 1 or 2.
